# EUROPEAN PATENT APPLICATION

(11) **EP 1 749 487 A2**
(43) Date of publication of application: **07.02.2007**
(21) Application number: 06076401.6
(22) Date of filing: 12.07.2006
(51) Int. Cl.: A61B 17/16

(54) **Surgical tool for scraping and collecting bone particles**

(30) Priority: 05.08.2005 IT RE20050098
(71) Applicant: C.G.M. S.P.A., 42015 Correggio (Reggio Emilia) (IT)
(72) Inventor: Parmigiani, Corrado Saverio, 42015 Correggio (Reggio Emilia) (IT)
(74) Representative: Corradini, Corrado

(57) **Abstract**

The tool comprises a handle (10) which bears a scraping blade (30) placed at the front end of the handle (10) and adapted to scrape particles from a bone, a collection chamber (20) of the removed material inside the front end portion of the handle (10), and a passage opening (21) in the collection chamber (20), placed near the scraping blade (30), which permits the passage of the scraped particles within the chamber (20). According to the invention, the collection chamber (20) has nearly cylindrical form and the tool comprises a collection means (40) of the particles having tubular form, insertable and extractable from the collection chamber (20) itself. The collection means (40) comprises a tubular covering (41), adapted to contain the bone particles scraped from the blade (30), whose front end portion (41') has a section adapted to substantially occupy the entire section of the collection chamber (20), for collecting the particles from the chamber (20) itself.

## Description

The present invention regards the techniques of reconstructive and regenerative removal of the bone tissues in oral-maxillofacial, plastic, periodontal and implant orthopaedic surgery techniques as well as plastic surgery techniques on bone.

Recently, tools have been developed for harvesting blocks or cylinders of bone in various zones of the skeletal structure and for working and manipulating the blocks with the object of obtaining granules or particles of suitable size for tissue-regenerative biological needs.

Such tools have permitted increasing the autologous bone harvesting techniques and the treatment itself of the bone material harvested, capable of obtaining bone granules for filling bone defects or for increasing skeletal structures. In order to make the harvesting, collection and grinding process simpler and quicker, decreasing the post-surgery discomfort of patients, the harvesting methods have been refined in the last few years with the introduction on the market of tools for removing and collecting bones particles (shavings or chips) by means of scraping, which comprise a handle having a scraping blade placed at the front end of the handle, and a collection chamber of the removed material foreseen in its front end.

Said devices permit the removal of surface cortical bone by means of a particular scraping blade, which the tool is equipped with, which generates bone particles in the form of thin shavings or chips, which are directly collected inside the collection chamber.

The high technology of the scraping blade permits obtaining, with a light pressure, an optimal and controllable cutting. The collection of the scraped bone particles occurs by means of a passage opening (slit) placed at the base of the blade which conveys the particles inside the collection chamber defined by a suitable protected receiver for temporary storage. In the collection step, the bony particles are mixed with the blood to form a high bone density concentrate, ideal as filler in regenerative techniques.

With a simple sliding operation, one obtains the opening of the collection chamber which contains the previously removed bone material, rendering it immediately available to be deposited in the recipient site.

One of said tools is illustrated in the patent US 5,683,406; here the tool comprises a blade in the form of a sliding plate which closes the cavity of a handle, which also functions as chip collector. A half-moon shaped opening is made on the blade, whose side defines a cutting edge for scraping the bone which appears on the side of the plate which is outside said cavity and remains accessible from the outside in any blade position.

The chip is created by placing said cutting edge in contact with the donor bone, and making it slide along the same by keeping it pressed with a certain force.

This type of tool does not lend itself to being made with relatively small cross sections.

Another tool, illustrated in EP 1405602-A of the same Applicant comprises a handle which bears a scraping blade at its front end and possesses a cylindrical or nearly cylindrical collection chamber formed by an outer covering, also functioning as a handle; a longitudinal stem is placed inside the collection chamber at whose front end the scraping blade is placed. This tool is capable of making the cross section of the tool a certain amount smaller, so to make the harvesting technique minimally invasive.

One object of the present invention is to perfect this type of tool in order to perfect, simplify and make more effective the harvesting of the particles from the collection chamber and transferring them to the recipient site in a quick and aseptic manner, eliminating further manipulations.

Another object is to make a tool whose front portion can have the smallest cross section possible, so to make the harvesting technique minimally invasive.

Another object is to realise a tool whose operation can be better controlled by the operator.

Another field of use of the present invention is in the plastic surgery of the bones, where an osteotome is currently used for the bone corrections and/or corrective bone removals. Such tool is commonly found on the market in different surgical disciplines: dental, maxillo, orthopaedics etc. The production material is steel of various hardness, which can therefore be resterilised; wear occurs after various uses, but it is possible to resharpen them with surgical stones.

The tool is particularly used in nose surgery for the removal of bumps and/or bone projections, also lateral, of the bone septum with the aid of the hammer. In such cases, there is the danger of not very high precision and the dangerous control of the tool by the operator in endoscopic tunnel techniques. The damage to the patient due to the hammer blows is also considerable, creating edemas (swelling) and ecchymoses (bruises) in the post-surgery course.

Alternatively, the rhinoplastic operation often employs bone-removing tools called raspatories, they too in steel like the osteotome and can be resterilised, and are already used in different surgical areas. They nevertheless have more or less invasive sizes, and teeth which permit a gradual removal over the entire working area of the raspatory; hence this is a not perfectly defined removal.

With reference to this tool type, another object of the invention is to realise a tool capable of better controlling the removal of the chips in relation with the aesthetic requirements of the tissues.

Said and other objects are achieved by the invention at hand as characterised in the claims.

Due to the present invention, it is possible to realise a convenient and safe (regarding its sterility and asepsis) transfer of the collected particles to the recipient site, especially in cases of relatively narrow and deep cavities.

It is moreover possible to realise a tool having the entire handle and above all the front portion made of one piece; due to this it is possible to realise a relatively long and narrow front portion in order to be able to carry out surface cortical bone harvesting in narrow areas, as an example (but not only) by means of interstitial tunnelling in various structures of the oral-facial system, with the object of considerably reducing the post-operation discomfort of patients and speeding up such removal techniques.

Preferably, the tool handle is composed of synthetic resin adapted for medical use, such as for example polycarbonate or acrylonitrile/butadiene/styrene.

The invention is set forth in detail below with the aid of the attached figures, which illustrate one of its embodiments as a non-exclusive example.
Fig. 1A is a rear and top perspective view of an embodiment of the tool according to the invention, wherein the collection means 40 are in a position partially inserted in the handle 10.

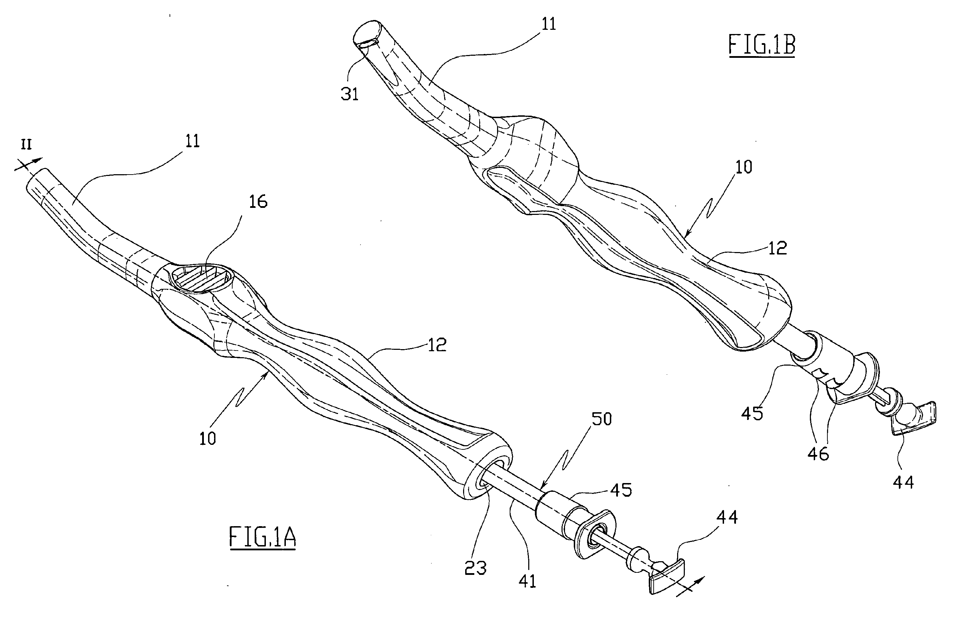

Fig. 1B is a front and bottom perspective view of Fig. 1A.
Fig. 2 is a section along a vertical axial plane of Fig. 1A, wherein the collection means is completely inserted in the handle 10.
Fig. 2A is the same section of Fig. 2, where the collection means is in extracted position from the tool handle.
Fig. 3 is an enlarged detail of Fig. 2.
Fig. 4 is an enlarged detail of Fig. 3, where the tool is in use.
Fig. 4A is a front view of Fig. 4.
Fig. 5 is the front view of Fig. 2.
Fig. 6 shows the use of the collection means in the transfer step of the collected particles to the recipient site.
Fig. 7 shows an alternative embodiment of the front end of the tool.
Fig. 7A is a front view of Fig. 7.
Fig. 8 shows a further alternative embodiment of the front end of the tool.
Fig. 8A is a front view of Fig. 8.
Fig. 9 is an enlarged detail of the right part of Fig. 2.
Fig. 10 is an enlarged detail of the left part of Fig. 2. The illustrated tool in the figures comprises a long, narrow handle 10 adapted to be held by the user's hand which bears a scraping blade 30, having a cutting edge 31 adapted to scrape particles from a bone, placed at the front end of the handle 10, i.e. the more distant end from the user's hand.

The handle 10 possesses a front end portion 11 having a relatively small diameter and a rear portion 12 shaped so to make its grip by the user's hand more effective.

In particular, the blade 30 is formed by a thin disc and is substantially circular, placed on a plane which is orthogonal to the axis of the front end portion 11, and above all has a circular arc cutting edge 31 which projects somewhat with respect to the surface of the handle in the zone surrounding the edge itself.

The tool moreover possesses a collection chamber 20 wherein the material removed from the bone is collected, realised inside the front end portion 11 of the handle, having cylindrical or nearly cylindrical shape (i.e. having constant section) and having rectilinear or curved axis. Its section can be of various form, but is preferably rounded and in particular circular. At the starting end of the chamber 20, an opening 21 is placed for the passage of the particles in the collection chamber 20. The opening 21 is realised on the end of the front end portion 11, in a lower position, placed near the cutting edge 31 of the blade 30, which permits the passage of the scraped particles within the chamber 20 itself.

In particular, the passage opening 21 has the form of a rectangular groove made on the end edge of the front edge portion 11, facing the cutting edge 31.

The tool according to the invention comprises a collection means 40 of the particles having tubular shape, insertable and extractable from the collection chamber 20 to collect the particles from the chamber 20 itself.

The collection means 40 comprises a tubular covering 41 adapted to contain the bone particles scraped by the blade 30, whose front end portion 41' has a cross section adapted to substantially occupy the entire transverse section of the collection chamber 20.

In particular, the collection chamber 20 possesses a constant or nearly constant section or rounded form (preferably circular) and the tubular covering 41 of the collection means possesses a corresponding constant or nearly constant section of equally rounded form adapted to adhere to the inner surface of the collection chamber 20.

Moreover, the front end portion of the covering 41 is adapted to scrape against the inner surface of the collection chamber 20, to collect the particles present in the chamber itself. In particular, the front end edge 41a of the covering 41 is relatively thin and clearly defined in order to realise an edge capable of separating the particles from the inner surface of the chamber 20 and to collect them inside the covering 41 itself.

The collection means 10 comprises a plunger 42 sliding within the tubular covering 41, adapted to expel the collected bone particles from the covering 41; in particular, the plunger 42 sealingly adheres to the inner surface of the tubular covering 41 so to realise an action capable of sucking the bone particles and dragging them inside the front part of the covering 41.

The plunger 42 is actuated by a thin stem 43, to whose end it is fixed; the rear end 44 of the stem exits outward from the rear end of the covering 41.

The front end portion 11 of the handle is a single piece and has substantially circular shape and substantially constant section. The collection chamber 20 is made inside the portion 11; in the chamber 20 the bone particles scraped from the blade 30 enter and are collected. The chamber 20 extends in a continuous manner towards the rear, giving place to a second rear chamber 22, in particular with rectilinear axis (in which the bone particles do not normally reach), in particular having substantially the same section as the chamber 20, at least in the front segment. The chamber 22 leads to the outside through an opening 23 in the handle placed in the rear position, adapted to permit the insertion and extraction of the collection means 40 from the collection chamber 20.

At the rear end of the tubular covering 41, a body 45 of plug form is fixed which is axially crossed by the covering 41 and is adapted to close the rear opening 23 like a plug. When the body 45 is inserted within the opening 23, the entire covering 41 is placed inside the handle 10 and the front end of it is placed close to the front end of the collection chamber 20. The rear end 44 of the stem 43 instead projects outside the handle 10 and covering 41.

The rear body 45 also serves for grasping in the easiest manner the covering 41 between two fingers when the collection means 40 are gripped, in the manner of a syringe, to expel its contents (as will be described below in greater detail).

The entire handle 10 and in particular its front end portion 11 are monolithic, i.e. they are made from a single piece or from several pieces integrally and fixedly joined together.

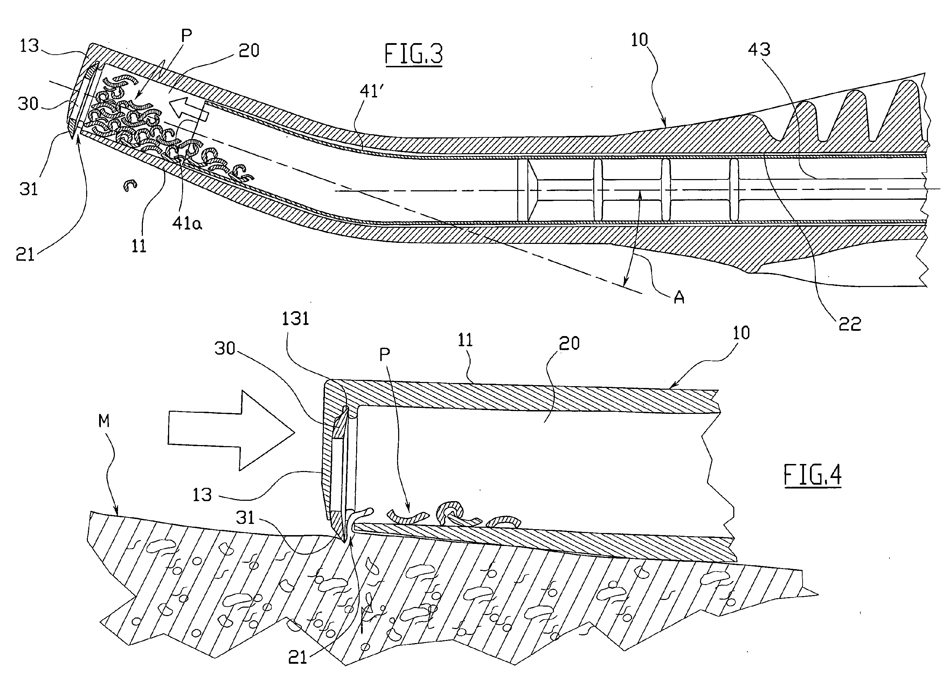

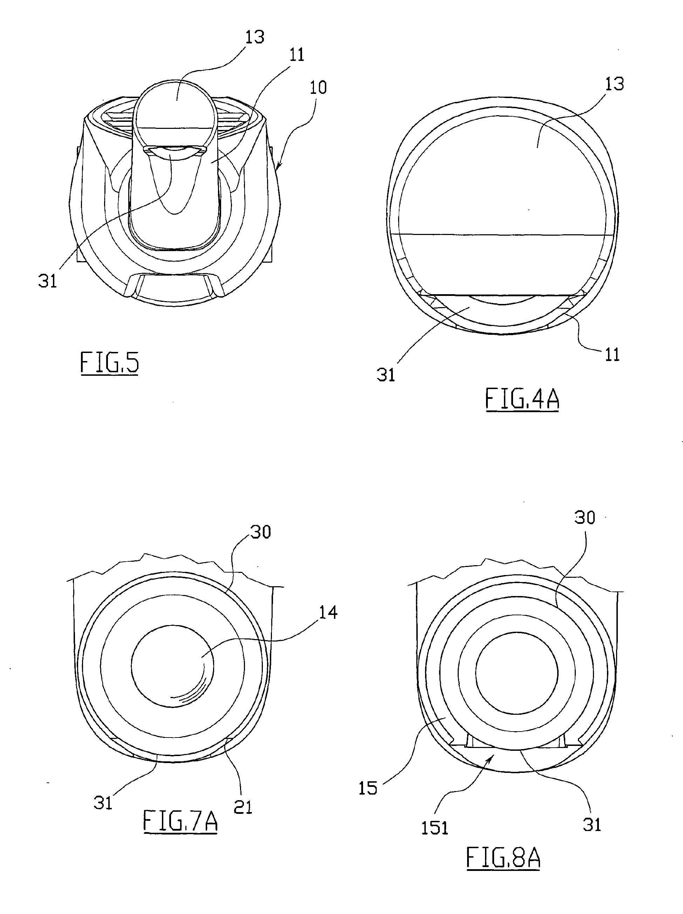

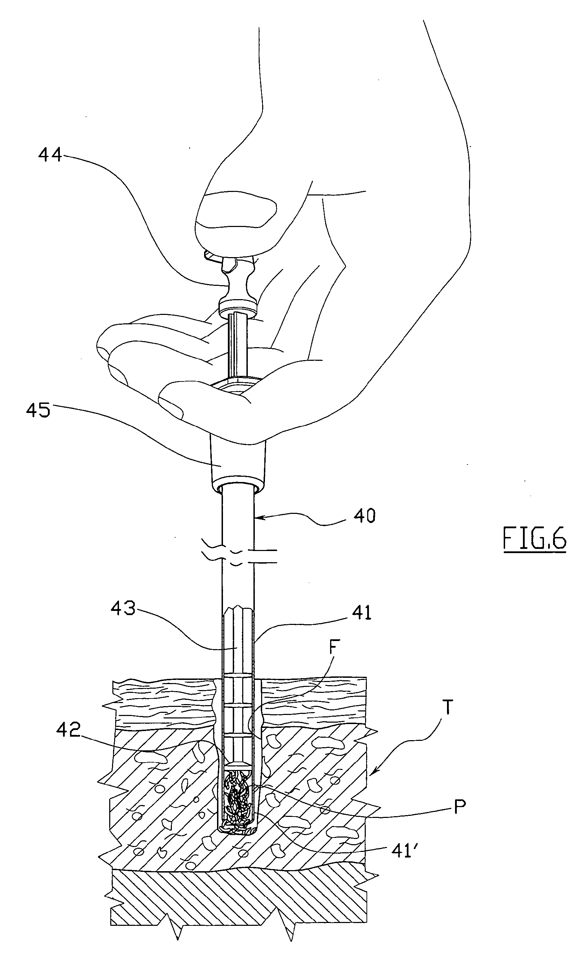

This consequently implies that its structure is stiffer and thus the front end portion 11 can in particular be realised with relatively small diameter. The rear portion 12 of the handle has ergonomic form to best adapt it to handling by the user. In particular, in addition to having a general form with variable section, it has a shaped portion 16, provided with teeth and turned upwards, adapted to receive the user's thumb in abutment. In the figures, the entire handle is illustrated as a single monolithic body, even if in reality, for its moulding it is preferable that two (or more) complementary elements are independently formed, which are subsequently integrally fixed to each other. For example, one of these components of the handle can be realised with a transparent resin, so to be able to visibly control the content of the collection chamber 20. In use, the tool, lacking the collection means 40, is first used for scraping and collecting bone particles with traditional methods. The cutting edge 31 of the blade 30 is made to slide along the surface of the bone M while the axis of the handle 10 is held at a small angle with respect to the surface of the bone M and is at the same time pressed against the bone itself. Following such action, the cutting edge 31 separates and raises the particles P from the bone M and moreover pushes them towards the inside of the collection chamber 20 through the opening 21 (see Fig. 4).

Once a quantity of bone particles is collected inside collection chamber 20, the collection means 40 are inserted through the rear opening 23, within the corridor formed by the rear chamber 22 and by the collection chamber 20. The tubular covering 41 is completely inserted within said corridor 20, 22 so to bring its front end portion 41' to the front end of the chamber 20; the front end of the covering 41 adheres to the inner surface of the chamber 20 for its entire section and therefore, while it advances along the chamber itself, its edge 41A slides, adhering to the inner surface of the chamber 20 and effectively collecting all of the bone particles P placed in the chamber itself. Of course, it is necessary in this step that the plunger 42 is moved back a distance from the front end edge 41a so to define, inside the covering 41, sufficient space to contain the bone particles P collected inside the chamber 20.

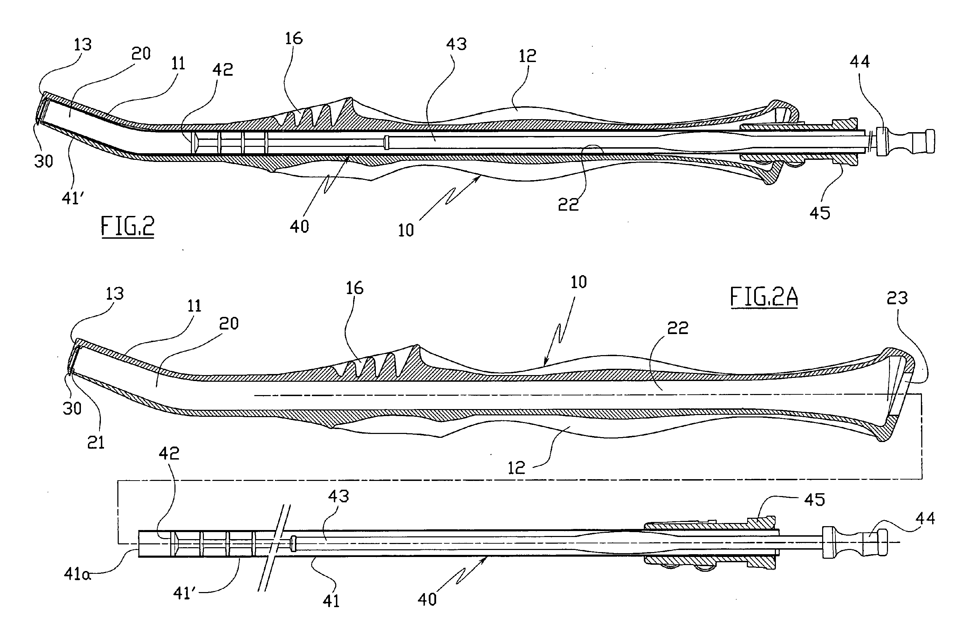

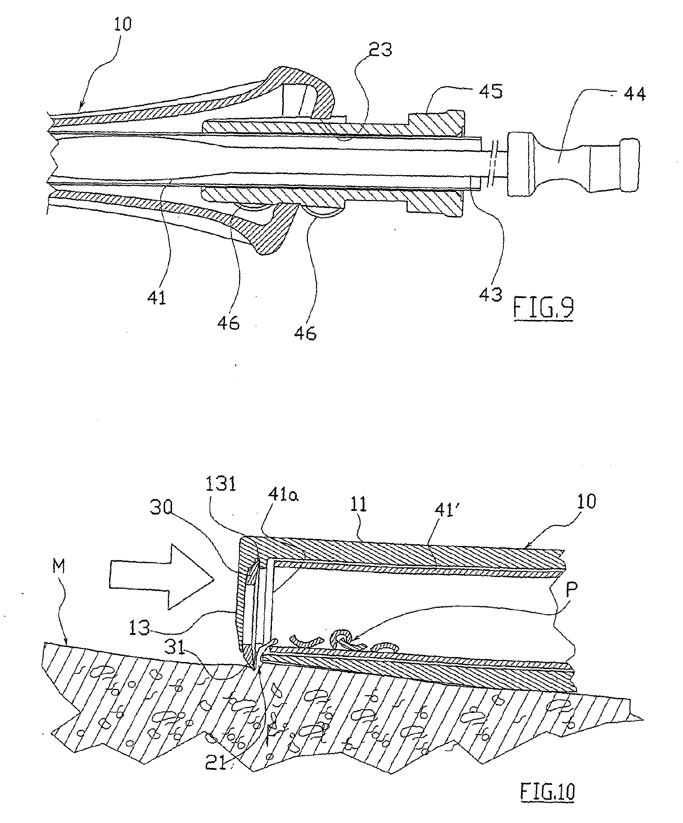

Alternatively, the scraping of the bone can be carried out with the collection means 40 stably inserted within the handle 10 (as illustrated in Fig. 2). The front portion 41' of the tubular covering 41 is placed within the collection chamber 20 with its front end edge 41a a short distance from the passage opening 21 so to not obstruct such opening (Fig. 10). Preferably, relief elements 46 are placed on the rear body 45 (Fig. 9), snapping with the edge of the rear opening 23 and adapted to make this position of the collection means 40 stable and precise within the handle 10.

In use, the particles P scraped from the bone, entering in the collection chamber are brought directly within the front end portion 41' of the covering 41, which occupies the chamber 20 (Fig. 10).

Once collected within its own front portion, the particles P present in the chamber 20, the collection means 40, and in particular the covering 41, possibly after having been pushed ahead until they have brought their own front edge 41a against the end of the collection chamber 20, or rather against the inner surface of the blade 30 (this movement is permitted due to the fact that the body 45 is capable of further penetrating through the opening 23), are pulled back and extracted through the same rear opening 23.

Once the bone particles are collected within the front zone of the covering 41, by operating on the rear end 44 of the stem 43 one may push the collected particles P within the covering 41, outside of the covering itself, by means of the plunger 42.

The collection means 40 optimally lends themselves to being used as a kind of syringe for directly inserting the collected material within the recipient site; this is particularly advantageous in the case wherein it is necessary to pour the particles P into narrow sites, for example within a hole F made in a bone tissue T of the same donor (see Fig. 6); due to the form of the means 40, the material P contained in it can effectively and conveniently be poured in the cavity of the hole F, also deeply.

Another advantage is that the material P passes directly from the collection chamber 20 to the hole P, without being further manipulated, and thus entirely advantageous for the asepsis and sterility conditions.

According to the embodiment illustrated in the figures, in order to make the use of the tool more practical and convenient inside the oral cavity in particular, the front end portion 11 of the handle 10 has a curved axis which defines an acute angle A between the front end portion 11 of the handle and the rear part of the same. In such case, the cutting edge 31 can be placed on the side of the portion 11 opposite the concavity formed by the axis curve (as illustrated in the figures), or on the side turned towards such concavity.

In such case, the collection means 40 is realised with flexible material so to be adapted to bend with respect to its longitudinal axis, to adapt itself to said possible curving of the chamber 20.

In the embodiment illustrated in Fig. 4 and 4A, the handle comprises, at its front end, an end wall 13 placed orthogonal to the final portion of the axis of the collection chamber 20, which frontally closes the chamber 20 itself, defining its front end. The blade 30 is formed by a thin disc which has the form of a circular, nearly flat crown and is inserted adjacent to the inner surface of said end wall 13. In detail, the circular edge of the blade 30 is inserted in an annular groove 131 placed along the periphery of the wall 13.

The edge of the blade 30 (which, in particular, is substantially circular) projects for a brief section of arc with respect to the wall 13, and respect to the lateral surface of the front end portion 11 near the passage opening 21, defining the cutting edge 31 along such arc with which the bone is scraped.

The fixing of the blade 30 to the wall 13 can be obtained by means of paste or other known fixing means.

Also in the second embodiment illustrated in Fig. 7 and 7A, the handle comprises, at its front end, an end wall 13 placed orthogonal to the final portion of the axis of the collection chamber 20, which frontally closes the chamber 20 itself. In this case, the wall 13 comprises an outer axial, prominent, and cylindrical spur 14 placed along the axis of the chamber 20, on which the blade 30 is perfectly fit; the blade 30, also in this case, is formed by a thin disc which has the form of a nearly flat circular crown.

Subsequently, the spur 14 is heat deformed and axially flattened towards the wall 13 so to assume the shape of a mushroom (illustrated with dashed lines in Fig. 7) whose head 14' is wider than the central hole of the blade 30 and locks the blade itself against the outer surface of the wall 13.

Here too, the edge of the blade 30 (which, in particular, is substantially circular) exits for short arc section with respect to the wall 13, and in general with respect to the lateral surface of the front end portion 11 near the passage opening 21, defining along such arc the cutting edge 31 with which the bone is scraped.

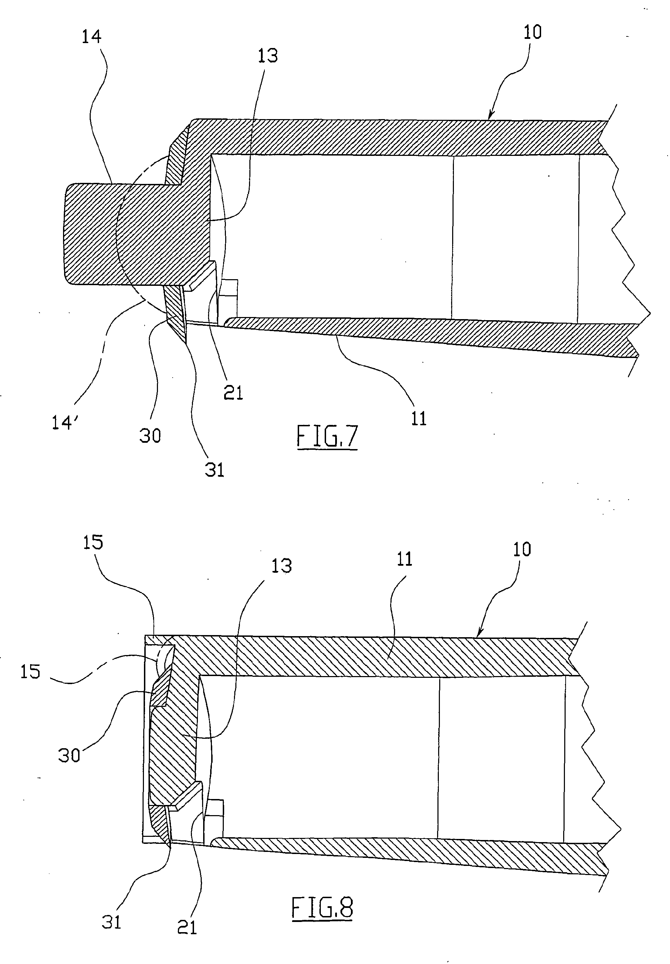

Also in the third embodiment illustrated in Fig. 8 and 8A, the handle 10 comprises, at its front end, an end wall 13 placed orthogonal to the final portion of the axis of the collection chamber 20, which frontally closes the chamber 20 itself.

In this case, the wall 13 comprises a side 15 in axial, outward relief and with circular arc longitudinal extension, which runs along the entire perimeter of the wall 13 except along a section of arc 151 of this, which is less than 180 degrees. The blade 30 also in this case has a circular form in plan view and is inserted in the space defined by the side 15; the side 15 is then heat deformed and radially folded towards the centre of the wall 13 (illustrated with the dashed line in Fig. 8) so to be folded over the outer surface of the blade and to lock the blade against the wall 13.

The edge of the blade 30 is substantially circular, exiting out of and projecting along said arch 151 with respect to the wall 13 and in general with respect to the lateral surface of the front end portion 11, defining along such arc the cutting edge 31 with which the bone is scraped.

Of course, numerous modifications of practical-applicative nature can be made to the invention at hand, without departing from the scope of the inventive idea as claimed below.

## Claims

1. Surgical tool for scraping and collecting bone particles, comprising a handle (10) which bears a scraping blade (30) placed at the front end of the handle (10) and adapted to scrape particles from a bone, a collection chamber (20) of the removed material inside the front end portion of the handle (10), and a passage opening (21) in the collection chamber (20) placed near the scraping blade (30), which permits the passage of the scraped particles within the chamber (20) itself,
**characterised in that**:
the collection chamber (20) has nearly cylindrical form and the tool comprises a collection means (40) of the particles having tubular form, insertable and extractable from the collection chamber (20) for collecting the particles from the chamber (20) itself.

2. Tool according to claim 1, **characterised in that** the collection chamber (20) communicates with an opening in the handle (10) placed in rear position, adapted to permit the extraction of the collection means (40) from the collection chamber (20).

3. Tool according to claim 1, **characterised in that** the collection means (40) comprises a tubular covering (41), adapted to contain the bone particles scraped by the blade (30), whose front end portion (41') has a section adapted to substantially occupy the entire section of the collection chamber (20).

4. Tool according to claim 1, **characterised in that** the collection means (40) comprise a covering (41) adapted to contain the bone particles scraped by the blade (30), whose front end portion (41') is adapted to scrape against the entire surface of the collection chamber (20), to collect the particles present in the chamber (20) itself.

5. Tool according to claim 3 or 4, **characterised in that** the collection means (40) comprise a plunger (42) sliding within the tubular covering (41) and adapted to expel the collected bone particles from the means themselves.

6. Tool according to claim 5, **characterised in that** the plunger (42) adheres to the inner surface of the tubular covering (41) to realise a suction action of the particles.

7. Tool according to claim 3, **characterised in that** the collection chamber (20) possesses a constant or nearly constant section and the tubular covering (41) of the collection means (40) possesses a corresponding constant or nearly constant section adapted to adhere to the inner surface of the collection chamber (20).

8. Tool according to claim 7, **characterised in that** the front end portion (11) of the handle (10) is made of a single piece, and has substantially circular form and substantially constant section, and inside of which at least the front end portion of the collection chamber (20) is made in coaxial position.

9. Tool according to claim 8, **characterised in that** the front end portion (11) of the handle (10) has a curved axis which defines an acute angle (A) between the front end of the handle (10) and its rear part.

10. Tool according to claim 5, **characterised in that** the collection means (40) is adapted to bend with respect to its longitudinal axis to adapt itself to possible curving of the axis of the front end portion (11) of the handle (10).

11. Tool according to claim 1, **characterised in that** the handle (10) comprises, at its front end, an end wall (13) which frontally closes the chamber (20) itself and the blade (30) is inserted adjacent to the surface of said end wall (13).

12. Tool according to claim 1, **characterised in that** the handle (10) comprises, at its front end, an end wall (13) which frontally closes the chamber (20); the wall (13) comprises an axial and prominent outer spur (14) placed along the axis of the chamber (20) and the blade (30) is formed by a thin disc of circular crown form which is inserted on said spur (14), said spur (14) being heat deformed and axially flattened towards the wall (13) so to define a type of mushroom which locks the blade (30) itself against the outer surface of the wall (13).

13. Tool according to claim 1, **characterised in that** the handle (10) comprises, at its front end, an end wall (13) which frontally closes the chamber (20); the wall (13) comprises a side (15) in axial, outward relief and with circular arc longitudinal extension, blade (30) having a circular form in plan view and being inserted in the space defined by the side (15), which is heat deformed and radially folded towards the centre of the wall (13) so to result folded onto the outer surface of the blade (30), locking it against the wall (13).
